# DEMANDE DE BREVET EUROPEEN

(11) **EP 4 169 554 A1**
(43) Date de publication de la demande: **26.04.2023**
(21) Numéro de dépôt: 22202887.0
(22) Date de dépôt: 21.10.2022
(51) Int. Cl.: A61M 5/315

(54) **SERINGUE INTÉGRANT UNE PUCE RFID**

(30) Priorité: 25.10.2021 FR 2111311
(71) Demandeur: France Developpement Electronique, 67700 Monswiller (FR)
(72) Inventeur: SCHAEFFER, Célestine, 67500 HAGUENAU (FR); JAOUEN, Gwenaël, 67300 SCHILTIGHEIM (FR); VICO, Raphaël, 67410 ROHRWILLER (FR)
(74) Mandataire: Cabinet Nuss

(57) **Abrégé**

La présente invention a pour objet une seringue (1) comprenant au moins un cylindre (2) et un piston monté mobile à l'intérieur du cylindre (2) en association avec une tige (31) d'actionnement, caractérisée en ce que l'extrémité de la tige (31) du piston opposée au cylindre (2) est associée à au moins une puce RFID par l'intermédiaire d'un logement configuré pour recevoir la puce RFID.

## Description

La présente invention se rapporte au domaine des systèmes d'identification des dispositifs d'injection de type seringue et plus particulièrement aux dispositifs d'identification intégrés à une seringue.

Dans le cadre de son utilisation, notamment dans le milieu médical, une seringue fait l'objet de plusieurs manipulations successives qui nécessite souvent une traçabilité. En effet, les différentes manipulations de la seringue sont susceptibles d'être effectuées par plusieurs intervenants. Ainsi, le stockage de la seringue, le remplissage de son réservoir par une solution injectable, l'opération d'injection, voire même sa surveillance, peuvent être réalisées par une chaine d'intervenants successifs : pharmacie, préparateur, infirmier, médecin qui sont parfois contraints à être dans l'incapacité de communiquer directement certaines informations rattachées l'injection et au dispositif utilisé.

Aussi, pour s'affranchir de cette contrainte, une identification des seringues a été développée. Dans un premier temps, cette identification a été réalisée de façon manuscrite sur une étiquette fixée sur la paroi du réservoir de la seringue puis, par la suite, un nouveau type de seringue a été développé en y intégrant une puce RFID. Dans le cadre de cette évolution, différentes seringues équipées de puce RFID ont été proposées avec une intégration de l'élément d'identification dans la tête du piston. Cette solution permet d'associer la puce RFID à la seringue en profitant de la différence de matériaux susceptible d'exister entre différents éléments constitutifs du mécanisme d'injection que sont, d'une part, la tête en extrémité du piston et, d'autre part, sa couverture élastique destinée à étanchéifier le déplacement du piston à l'intérieur du réservoir de la seringue. Toutefois, une telle solution présente comme inconvénient d'intégrer la puce RFID au niveau d'une zone critique de la seringue. En effet, lors d'une utilisation du dispositif d'injection en milieu médical, il est indispensable que la tête du piston en déplacement à l'intérieur du réservoir de la seringue ne soit pas sujet à une contamination. Aussi, le positionnement d'une puce RFID dans la tête du piston impose une étape d'intégration de la puce à une pièce de la seringue préalablement à l'assemblage de l'ensemble des éléments qui forment la seringue. De plus, en cas de puce défectueuse, celle-ci n'est pas en mesure d'être remplacée sans altération du réservoir de la seringue.

La présente invention a pour but de pallier ces inconvénients en proposant un dispositif d'injection de type seringue qui permette un suivi et une traçabilité de ce dispositif par intégration d'une puce RFID sans impacter la construction de cette seringue tout en autorisant un éventuel remplacement de cette puce RFID lorsque celle-ci présente un défaut.

L'invention a ainsi pour objet une seringue comprenant au moins un cylindre et un piston monté mobile à l'intérieur du cylindre en association avec une tige d'actionnement, caractérisée en ce que l'extrémité de la tige du piston opposée au cylindre est associée à au moins une puce RFID par l'intermédiaire d'un logement configuré pour recevoir la puce RFID.

L'invention concerne aussi un dispositif de fabrication d'une seringue selon l'invention, caractérisé en ce que le dispositif de fabrication comprend :
- une interface de chargement d'au moins une puce RFID,
- un mécanisme d'insertion en translation comprenant une interface de poussée d'une puce RFID depuis l'interface de chargement vers le logement d'une seringue selon l'invention,
- une interface de réception de l'arrangement structurel d'extrémité d'une seringue selon l'invention, configurée pour orienter l'ouverture d'insertion en direction et de niveau avec le mécanisme d'insertion.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :

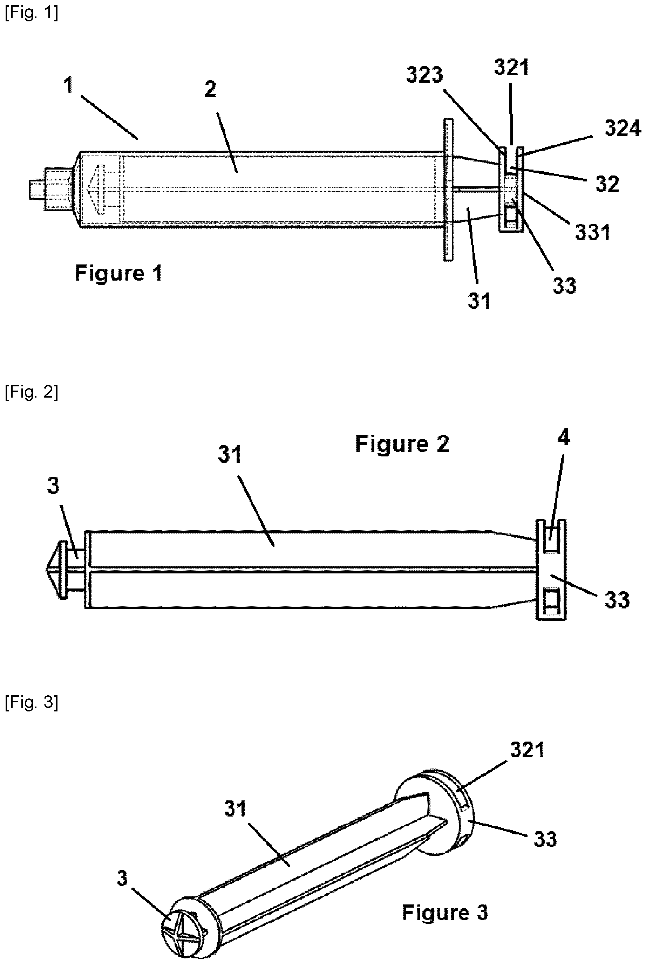

[Fig. 1] représente une illustration schématique d'un exemple de seringue selon l'invention apte à recevoir une puce RFID,
[Fig. 2] représente une illustration schématique selon une vue latérale d'un exemple de tige d'actionnement pour seringue selon l'invention intégrant une puce RFID,
[Fig. 3] représente une illustration schématique d'un exemple de tige d'actionnement pour seringue selon l'invention apte à recevoir une puce RFID,

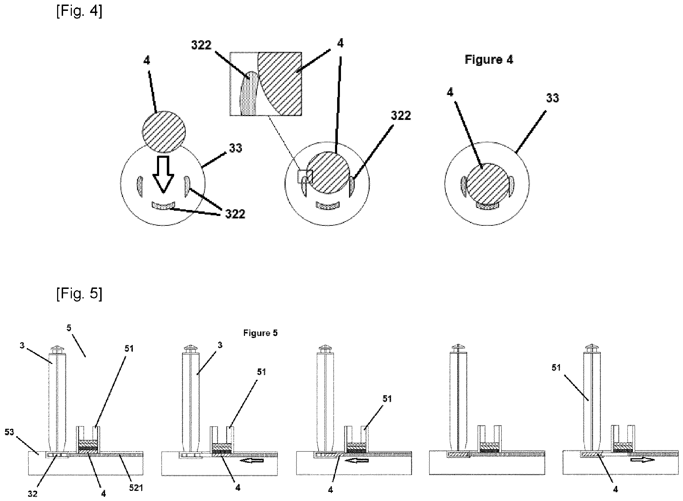

[Fig. 4] représente une illustration schématique selon une section perpendiculaire à l'axe d'une tige d'actionnement de seringue d'un exemple de positionnement d'une puce RFID,
[Fig. 5] représente une illustration schématique selon une vue latérale d'un exemple d'insertion d'une puce RFID en extrémité d'une tige d'actionnement de seringue.

L'invention concerne une seringue 1 comprenant au moins un cylindre 2 et un piston 3 monté mobile à l'intérieur du cylindre 2 en association avec une tige 31 d'actionnement, caractérisée en ce que l'extrémité de la tige 31 du piston 3 opposée au cylindre 2 est associée à au moins une puce RFID 4 par l'intermédiaire d'un logement 32 configuré pour recevoir la puce RFID 4. Le positionnement de la puce RFID 4 au niveau de l'extrémité du piston 3 positionnée vers l'extérieure de la seringue 1 réalise un arrangement où l'accès à la seringue 1 est facilité pour y intégrer la puce 4. L'intégration de la puce RFID 4 à la seringue 1 est ainsi détachée de toute contrainte liée à la position de la tête du piston 3 en insertion à l'intérieur du cylindre 2. Aussi, le positionnement de la puce RFID 4 à l'intérieur d'un logement 32 dédié porté par l'extrémité de la tige 31 du piston 3 est susceptible d'être effectuée préalablement à l'assemblage du piston 3 avec le cylindre 2 ou, alternativement, postérieurement à cet assemblage. Ainsi, l'intégration de la puce RFID 4 à la seringue 1 est susceptible d'être réalisée sans que le contenu de la seringue 1 ou l'intérieur du cylindre 2 ne soit affecté, par exemple, par une éventuelle contamination.

Selon un exemple correspondant à une variante de construction de la seringue 1 de l'invention, le logement 32 comprend une complémentarité de surface avec au moins une surface de la puce RFID 4. Cette complémentarité permet d'opérer une stabilisation de la puce RFID 4 positionnée à l'intérieur du logement 32 au niveau d'au moins une surface de contact. Selon un arrangement préféré de réalisation du logement 32 dans la seringue 1 de l'invention, celui-ci est réalisée de façon à réaliser une cavité dont le volume intérieur présente au moins une complémentarité partielle avec la forme de la puce RFID 4. Cette complémentarité est ainsi susceptible de limiter le déplacement ou les débattements de la puce RFID 4 à l'intérieur du logement 32. Si une telle mobilité de la puce RFID 4 dans son logement 32 n'a que peu d'impact sur la dégradation de son fonctionnement, en revanche elle est susceptible d'apporter une impression de fragilité de la seringue 1 à un manipulateur. La complémentarité de forme qui participe au blocage de la puce RFID 4 dans son logement 32 permet de supprimer cette sensation de fragilité.

Il convient de relever que le volume intérieur du logement 32 est susceptible d'être réalisation selon différents types de configurations : carré, rectangulaire, triangulaire, circulaire, disque, notamment aplati ou plus épaisse en forme de pavé, conique, etc. De façon préférée, la forme du volume intérieur du logement 32 est adaptée à la forme extérieure de la puce RFID 4. Une forme arrondie de la puce RFID 4 notamment circulaire telle un disque est préférée. En effet, l'insertion d'une puce RFID 4 ayant la forme d'un disque dans un logement 32 de forme au moins partiellement complémentaire, c'est-à-dire présentant un arrangement en partie semi-circulaire, n'impose aucune orientation particulière de la puce RFID 4 pour être positionnée correctement dans le logement 32.

Selon un autre exemple correspondant à une variante de construction de la seringue 1 de l'invention susceptible d'être combinée avec les variantes précédemment détaillées, l'extrémité de la tige 31 du piston 3 comprenant un arrangement structurel 33 selon lequel le logement 32 de la puce RFID 4 est réalisé dans l'épaisseur et selon l'axe de la tige du piston 31 de sorte que le logement présente au moins un orifice d'insertion de la puce RFID 4 ouvert vers l'extrémité de la tige 31 du piston 3 opposée au cylindre 2 de la seringue 1. L'intégration de la puce RFID 4 se trouve alors opéré par insertion selon un axe sensiblement parallèle, voire même coaxial, avec l'axe de la tige 3 du piston 31.

Selon un autre exemple correspondant à une variante de construction de la seringue 1 de l'invention formant une alternative à la variante précédente, l'extrémité de la tige 31 du piston 3 comprenant au moins une surface poussoir 331 portée par un arrangement structurel 33 d'extrémité disposé dans un plan sensiblement perpendiculaire à l'axe de la tige 31 du piston 3, le logement 32 de la puce RFID 4 est réalisé dans l'épaisseur de l'arrangement structurel 33 plan. Selon cette variante de construction, l'intégration de la puce RFID 4 à la seringue 1 profite d'un arrangement structurel d'une partie de la seringue 1 notamment de l'extrémité de la tige 31 du piston 3 pour le positionnement d'un logement 32 apte à recevoir une puce RFID 4. En l'espèce, la surface poussoir 331 réalise une interface en extrémité de la tige 31 du piston 3 au niveau de laquelle un utilisateur, voire éventuellement un dispositif mécanisé, exerce une pression afin de réaliser un déplacement du piston 3 vers l'intérieur du cylindre 2 de la seringue 1 dans le cadre d'une opération d'injection du contenu du cylindre 2. Cette surface poussoir 331 est classiquement portée par un arrangement structurel 33 qui présente une épaisseur suffisante pour rigidifier la surface poussoir 331 et éviter la déformation de cette dernière lorsqu'une pression est exercée contre celle-ci. Le logement 32 destiné à réceptionner la puce RFID 4 est réalisé dans l'épaisseur de cet arrangement structurel 33 d'extrémité. Ce positionnement permet de profiter d'une portion structurelle de la tige 3 de la seringue 1 qui soit suffisamment importante pour permettre le positionnement d'un logement 32. La rigidité de cet arrangement structurel 33 d'extrémité participe également à la protection de la puce RFID 4 en position à l'intérieur du logement 32. L'arrangement structurel 33 réalise ainsi une coquille de protection de la puce RFID 4.

Selon un exemple correspondant à une variante particulière de la variante de construction précédente, le logement 32 comprend au moins une ouverture 321 d'insertion positionnée au niveau d'au moins une portion périphérique de l'arrangement structurel 33 d'extrémité de la tige 31. Le logement 32 présentant un arrangement sensiblement plan orienté perpendiculairement à l'axe de la tige 31 du piston 3, une ouverture 321 positionné au niveau de la surface périphérique de l'arrangement structurel 33 permet une insertion de la puce RFID 4 selon un axe perpendiculaire à l'axe de la tige 31 du piston 3. Aussi, l'ouverture 321 se trouve positionnée au niveau d'une surface de l'arrangement structurel 33 d'extrémité de la tige 31 qui soit différente de la surface poussoir 331, de sorte que la puce RFID 4 se trouve protégée contre toute interaction parasite lorsqu'une pression est exercée par un utilisateur, voire éventuellement un dispositif mécanisé, contre la surface poussoir 331 de la seringue 1. Le risque d'une dégradation de la puce RFID 4 par un utilisateur ou un dispositif mécanisé au travers de l'ouverture 321 d'insertion au cours de l'opération d'injection se trouve ainsi limitée. Selon un exemple de construction, l'ouverture 321 d'insertion présente une forme de fente adaptée à une puce RFID 4 de forme aplatie.

Selon un exemple correspondant à une variante spécifique de la variante particulière précédente, l'ouverture 321 d'insertion comprend au moins une portion de son bord périphérique qui porte une lèvre 322 flexible. Cette lèvre 322 est susceptible d'être positionnée au niveau d'une unique portion du bord de l'ouverture 321. Toutefois, de façon préférentielle, le bord de l'ouverture 321 comprend au moins deux portions disposées en vis-à-vis l'une de l'autre, de part et d'autre de l'ouverture 321 d'insertion. Cette lèvre 322 ou cette paire de lèvres 322 flexibles est ainsi susceptible d'être déformée lors d'une insertion de la puce RFID 4 à l'intérieur du logement 32 au cours de laquelle le bord de la puce RFID 4 appuie contre le bord de l'ouverture 321 d'insertion. Ainsi, dans un premier temps, Cette lèvre 322 ou cette paire de lèvres 322 flexibles se trouve écartée par rapport au centre de l'ouverture 321 d'insertion puis, une fois la puce RFID 4 insérée dans le logement 32, revient, au moins partiellement, en position.

Selon un exemple correspondant à une autre variante spécifique de la variante particulière précédente et susceptible d'être combinée avec la variante spécifique précédemment détaillée, dans un plan sensiblement perpendiculaire à l'axe de la tige 31 du piston 3, l'ouverture 321 d'insertion présente un dimensionnement inférieur à la plus grande dimension de la puce RFID 4 dans ce même plan. Ainsi, l'insertion de la puce RFID 4 à l'intérieur du logement 32 est opérée en force, de sorte que les bords de la puce RFID 4 entre en contact et exerce une pression contre des portions opposées du bord de l'ouverture 321 d'insertion. Aussi, lorsque le bord de l'ouverture 321 d'insertion comprend une ou plusieurs lèvres 322, la puce RFID 4 en insertion dans le logement 32 exerce une pression contre les lèvres 322 du bord de l'ouverture 321 d'insertion entrainant leur écartement puis, une fois la puce RFID 4 à l'intérieur du logement 32, les lèvres 322 retourne en position, au moins partiellement, vers le centre de l'ouverture 321 d'insertion. Par ailleurs, cette lèvre 322 ou cette paire de lèvres 322 flexibles en combinaison avec un dimensionnement de l'ouverture 321 d'insertion inférieur à la plus grande dimension de la puce RFID 4, est également susceptible de réaliser un moyen élastique apte à retenir la puce RFID 4 à l'intérieur du logement 32. En revenant en position vers le centre de l'ouverture 321 d'insertion, la lèvre 322 ou la paire de lèvres 322 effectue un cloisonnement partiel de l'ouverture 321 du logement 32 voire même, de façon complémentaire ou alternative, réalise un pincement de la puce RFID 4 en appuyant contre au moins un des bords de la puce RFID 4 pour maintenir cette puce RFID 4 en blocage à l'intérieur du logement 32. L'insertion et la retenue de la puce RFID 4 à l'intérieur du logement 32 par la lèvre 322 ou la paire de lèvres 322 est ainsi similaire à un mécanisme de clipsage de la puce RFID 4 à l'intérieur du logement 32.

Selon un autre exemple correspondant à une variante de construction de la seringue 1 de l'invention susceptible d'être combinée avec l'une ou l'autre des variantes précédemment détaillées, le logement 32 comprend une seconde ouverture positionnée au niveau d'au moins une portion périphérique de l'arrangement structurel 33 d'extrémité de la tige 31 et opposée à l'ouverture 321 d'insertion à l'intérieur du logement 32. Cette seconde ouverture est configurée pour permettre l'insertion au travers de celle-ci d'un élément extracteur, de sorte que l'élément extracteur soit en mesure d'appuyer contre une surface de la puce RFID 4 à l'intérieur du logement 32 pour que la puce RFID 4 soit éjecter du logement 32 au niveau de l'ouverture 321 d'insertion et dans un sens opposé à celui de l'insertion de la puce RFID 4 dans le logement 32.

Selon un autre exemple correspondant à une variante de construction de la seringue 1 de l'invention susceptible d'être combinée avec l'une ou l'autre des variantes précédemment détaillées, le logement 32 comprend au moins deux surfaces intérieures 323, 324 disposées dans des plans sensiblement perpendiculaires à l'axe de la tige 31 du piston 3 et présentant une inclinaison entre elles. Selon cette caractéristique particulière, le logement 32 présente deux surfaces intérieures 323, 324 opposées inclinées entre elles. Selon un premier exemple, cette inclinaison des surfaces 323, 324 réalise ainsi un angle susceptible d'être orienté de façon à être ouvert en direction de l'ouverture 321 d'insertion du logement 32. Aussi, lorsque la distance la plus réduite entre ces deux surfaces intérieures 323, 324 est inférieure à l'épaisseur de la puce RFID 4, une insertion de la puce RFID 4 à l'intérieur du logement 32 permet d'opérer un pincement de la puce RFID 4 entre ces deux faces 323, 324 opposées inclinées. Le sens de l'insertion de la puce RFID 4 présentant sensiblement la même orientation que celui du rapprochement des deux surfaces intérieures 323, 324 inclinées, la puce RFID 4 insérée dans le logement 32 est ainsi susceptible d'être coincée entre les deux surfaces 323, 324 opposées du logement 32. Selon un exemple de construction alternative à l'exemple précédent, l'inclinaison entre les deux surfaces intérieures 323, 324 réalise un angle orienté de façon à être fermé en direction et à proximité de l'ouverture 321 d'insertion du logement 32. Aussi, lorsque la distance la plus réduite entre les deux surfaces intérieures 323, 324 est inférieure à l'épaisseur de la puce RFID 4, une insertion de la puce RFID 4 au travers de l'ouverture 321 d'insertion du logement 32 est susceptible d'être effectuée en profitant de l'élasticité du matériau de l'arrangement structurel 33 d'extrémité. La puce RFID 4 inséré au travers de l'ouverture 321 d'insertion frotte et déforme les deux faces 323, 324 opposées inclinées, de sorte que l'inclinaison des faces 323, 324 opposées du logement 32 repousse la puce RFID 4 vers l'intérieur du logement 32 pour l'y maintenir.

L'invention concerne aussi un dispositif de fabrication 5 d'une seringue 1 selon l'invention, caractérisé en ce que le dispositif de fabrication 5 comprend :
- une interface de chargement 51 d'au moins une puce RFID 4,
- un mécanisme d'insertion en translation 52 comprenant une interface de poussée 521 d'une puce RFID 4 depuis l'interface de chargement 51 vers le logement 32 d'une seringue 1 selon l'invention,
- une interface de réception 53 de l'arrangement structurel 33 d'extrémité d'une seringue 1 selon l'invention, configurée pour orienter l'ouverture 321 d'insertion en direction et de niveau avec le mécanisme d'insertion 52.

L'interface de chargement 51 du dispositif de fabrication 5 réalise un volume de stockage de plusieurs puces RFID 4 destinée à être intégrées dans des seringues 1 respectives. Ces seringues 1 sont successivement positionnées au niveau de l'interface de réception 53, en vis-à-vis de l'interface de chargement 51 des puces RFID 4, de sorte que le mécanisme d'insertion en translation 52 opère un déplacement d'une puce RFID 4 dans le logement 32 de l'arrangement structurel 33 d'extrémité de la seringue 1.

Selon un exemple correspondant à une variante de construction du dispositif de fabrication 5 de l'invention, l'interface de chargement 51 est configurée pour recevoir au moins deux puces RFID 4 superposées, l'interface de poussée 521 du mécanisme d'insertion 52 étant configurée pour traverser l'interface de chargement 51 au niveau de la position de la puce RFID 4 destinée à être insérée. Ainsi, selon cet exemple de construction, les puces RFID 4 sont empilées à l'intérieur de l'interface de chargement 51, de sorte que le dispositif de fabrication 5 intègre dans le logement 32 dédié d'une seringue 1, la puce RFID 4 positionnée au niveau inférieur de la superposition. L'interface de chargement 51 comprend une paire d'orifices positionnés de part et d'autre de l'interface de chargement 51 au niveau de son extrémité inférieure, de sorte que ces deux orifices puissent être traversés par l'interface de poussée 521 du mécanisme d'insertion 52 selon un axe sensiblement perpendiculaire à l'axe de l'empilement des puces RFID 4 superposées à l'intérieur de l'interface de chargement 51. Ces orifices présentent des profils similaires, d'une part, à celui de la section de l'interface de poussée 521 et, d'autre part, à une section d'une puce RFID 4. La hauteur de ces orifices est donc supérieure à la hauteur d'une puce RFID 4 et au moins inférieure à la hauteur de deux puces RFID 4 superposées. Aussi, la translation de l'interface de poussée 521 au travers de l'interface de chargement 51 opère un déplacement de la puce RFID 4 positionnée au niveau inférieur de la superposition. Cette translation de l'interface de poussée 521 éjecte la puce RFID 4 de l'interface de chargement 51 pour la diriger vers l'ouverture 321 d'insertion du logement 32 dans l'arrangement structurel 33 d'extrémité de la seringue 1 positionnée au niveau de l'interface de réception 53. L'interface réception 53 d'une seringue 1 est ainsi configurée pour positionner le logement 32 porté par la seringue 1 de sorte qu'il se trouve de niveau avec l'interface de poussée 521 du mécanisme d'insertion 52. Une fois la puce RFID 4 intégrée à la seringue 1, l'interface de poussée 521 revient en position et sort de l'interface de chargement 51, de sorte que la superposition de puces RFID 4 se trouve décalée vers le bas par gravité pour occuper la position laissée vacante par la puce RFID 4 nouvellement intégrée à une seringue 1.

Il convient de relever que, de façon préférentielle, l'extrémité de l'interface de poussée 521 destinée à interagir avec un bord de la puce RFID 4 présente une forme sensiblement complémentaire du bord de la puce RFID 4. Idéalement, l'extrémité de l'interface de poussée 521 présente une concavité configurée pour loger au moins une partie de la puce RFID 4, de sorte que, lors de la translation de l'interface de poussée 521, la puce RFID 4 est maintenue associée à l'interface de poussée 521 et conserve son déplacement dans le logement 32 dans l'arrangement structurel 33 d'extrémité de la seringue 1.

Il convient également de relever que la course du déplacement de l'extrémité de l'interface de poussée 521 s'arrête préférentiellement au niveau, voire à proximité, de l'orifice d'insertion 321 du logement 32 porté par la seringue 1. Selon une variante particulière, l'extrémité de l'interface de poussée 521 termine sa course jusqu'à l'intérieur d'une partie du logement 32 porté par la seringue 1, de façon à assurer une insertion optimale, éventuellement en force, de la puce RFID 4 à l'intérieur du logement 32 dédié.

Selon un autre exemple correspondant à une variante de construction du dispositif de fabrication 5 de l'invention susceptible d'être combinée avec la variante précédemment détaillée, le dispositif de fabrication 5 comprend également une unité d'écriture d'une donnée sur la puce RFID 4 insérée. Selon la variante de positionnement de l'unité d'écriture dans le dispositif de fabrication 5, cette opération d'écriture est susceptible d'être opérée en amont ou en aval de l'intégration de la puce RFID 4 à une seringue 1.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Seringue (1) comprenant au moins un cylindre (2) et un piston (3) monté mobile à l'intérieur du cylindre (2) en association avec une tige (31) d'actionnement, **caractérisée en ce que** l'extrémité de la tige (31) du piston (3) opposée au cylindre (2) est associée à au moins une puce RFID (4) par l'intermédiaire d'un logement (32) configuré pour recevoir la puce RFID (4).

2. Seringue (1) selon la revendication 1, **caractérisée en ce que**, l'extrémité de la tige (31) du piston (3) comprenant au moins une surface poussoir (331) portée par un arrangement structurel (33) d'extrémité disposé dans un plan sensiblement perpendiculaire à l'axe de la tige (31) du piston (3), le logement (32) de la puce RFID (4) est réalisé dans l'épaisseur de l'arrangement structurel (33) plan.

3. Seringue (1) selon la revendication 2, **caractérisée en ce que** le logement (32) comprend au moins une ouverture (321) d'insertion positionnée au niveau d'au moins une portion périphérique de l'arrangement structurel (33) d'extrémité de la tige (3).

4. Seringue (1) selon la revendication 3, **caractérisée en ce que** l'ouverture (321) d'insertion comprend au moins une portion de son bord périphérique qui porte une lèvre (322) flexible.

5. Seringue (1) selon une des revendications 2 à 4, **caractérisée en ce que**, dans un plan sensiblement perpendiculaire à l'axe de la tige (31) du piston (3), l'ouverture (321) d'insertion présente un dimensionnement inférieur à la plus grande dimension de la puce RFID (4) dans ce même plan.

6. Seringue (1) selon une des revendications 2 à 5, **caractérisée en ce que** le logement (32) comprend au moins deux surfaces intérieures (323, 324) disposées dans des plans sensiblement perpendiculaires à l'axe de la tige (31) du piston (3) et présentant une inclinaison entre elles.

7. Seringue (1) selon une des revendications précédentes, **caractérisée en ce que** le logement (32) comprend une complémentarité de surface avec au moins une surface de la puce RFID (4).

8. Dispositif de fabrication (5) d'une seringue (1) selon une des revendications 2 à 7, **caractérisé en ce que** le dispositif de fabrication (5) comprend :
- une interface de chargement (51) d'au moins une puce RFID (4),
- un mécanisme d'insertion en translation (52) comprenant une interface de poussée (521) d'une puce RFID (4) depuis l'interface de chargement (51) vers le logement (32) d'une seringue (1) selon une des revendications 2 à 7,
- une interface de réception (53) de l'arrangement structurel (33) d'extrémité d'une seringue (1) selon une des revendications 2 à 7, configurée pour orienter l'ouverture (321) d'insertion en direction et de niveau avec le mécanisme d'insertion (52).

9. Dispositif de fabrication selon la revendication 8, **caractérisé en ce que** l'interface de chargement (51) est configurée pour recevoir au moins deux puces RFID (4) superposées, l'interface de poussée (521) du mécanisme d'insertion (52) étant configurée pour traverser l'interface de chargement (51) au niveau de la position de la puce RFID (4) destinée à être insérée.

10. Dispositif de fabrication selon une des revendications 8 ou 9, **caractérisé en ce que** le dispositif de fabrication (5) comprend également une unité d'écriture d'une donnée sur la puce RFID (4) insérée.
